# EUROPEAN PATENT SPECIFICATION

(11) **EP 1 808 188 B1**
(45) Date of publication and mention of the grant of the patent: **04.05.2011**
(21) Application number: 07405002.2
(22) Date of filing: 05.01.2007
(51) Int. Cl.: A61L 27/34, A61L 31/10, A61L 29/08

(54) **Cell selective implant surface with controlled release of bioactive agents**
Zellselektive Implantatoberfläche mit gesteuerter Freisetzung bioaktiver Wirkstoffe
Surface d'implantation sélective de cellules avec libération contrôlée d'agents bioactifs

(30) Priority: 11.01.2006 EP 06000453
(43) Date of publication of application: 18.07.2007
(60) Divisional application: 10012755.4
(73) Proprietor: Straumann Holding AG, 4002 Basel (CH); Eidgenössische Technische Hochschule Zürich, 8092 Zürich (CH)
(72) Inventor: Tosatti, Samuele, 8050 Zürich (CH); Trentin, Diana, 8004 Zürich (CH); Schuler, Martin, 8953 Dietikon (CH); de Wild, Michael, 4104 Oberwil (CH)
(74) Representative: Schaad, Balass, Menzl & Partner AG

(56) References cited:
- WO-A-03/072156
- WO-A2-2006/088368
- US-A1- 2002 128 234
- US-A1- 2003 099 682
- MÜLLER M. ET AL.: J BIOMED MATER RES, vol. 66A, 2003, pages 55-61, XP002419121
- TOSATTI S. ET AL.: "Peptide functionalized poly(l-lysine)-g-poly(ethylene glycol) on titanium: resistance to protein adsorption in full heparized human blood plasma" BIOMATERIALS, vol. 24, 2003, pages 4949-4958, XP002419122
- BODMEIER R.: "solvent selection in the preparation of poly(DL-lactide) microspheres prepared by the solvent evaporation method" INTERNATIONAL JOURNAL OF PHARMACEUTICS, vol. 43, 1988, pages 179-186, XP002419123
- GERMANIER Y. ET AL.: "enhanced bone apposition around biofunctionalized sandblasted and acid-etched titanium implant surface" CLIN. ORAL. IMPL. RES., vol. 17, 2006, pages 251-257, XP002419124

## Description

The present invention relates to an implant having a surface providing for controlled release of bioactive agents, according to claim 1, and to a kit for preparing the implant, according to claim 22.

Implantable artificial devices, such as artificial joints and teeth, play an important role in health care today. One of the major problems associated with implants is their biocompatibility. The better the biocompatibility is, the greater is the chance to avoid unwanted inflammatory processes. While inflammation may be at least partly reduced by the choice of proper materials, that is, materials which are biologically inert, other issues of implants known in the art, such as fast and mechanically stable osseo-integration remain to be solved satisfactorily.

Several ways to improve tissue/implant interaction have been described in the art:

US 2003/0099682 relates to an implant surface, comprising an implant and a bioactive polymer on at least a portion of the implant surface. The polymer layer contains at least one bioactive factor.

WO 03/072156 describes smooth and rough implant surfaces covered by a thin film of a graft-copolymer. The copolymer may be modified with peptides.

An enhanced osseo-integration was found after coating of titanium implants with RGD-functionalized PLL-g-PEG polymers.

Germanier, Y., et al., Enhanced Bone Apposition Around Biofunctionalized Sand-blasted and Acid-etched Titanium Implant Surfaces. A histomorphometric study in miniature pigs. Clinical Oral Implants Research, 2005. in press.

Tosatti, S., et al., RGD-containing peptide GCRGYGRGDSPG reduces enhancement of osteoblast differentiation by poly(L-lysine)-graft-poly(ethylene glycol)-coated titanium surfaces. Journal of Biomedical Materials Research, Part A, 2004. 68A(3): p. 458-472.

US 2002/0106345 relates to the local administration of anti-inflammatory drugs enhancing regeneration and healing of tissue. The anti-inflammatory drugs are incorporated in a polymer matrix.

US 5,876,454 relates to bioactive conjugates adapted to coat a metal implant surface, whereby the bioactive moiety promotes tissue growth.

WO 00/44305 relates to an implant made of titanium or titanium alloys with a roughened and hydroxylated surface.

Faraasen et al. (Pharmaceutical Research, Vol. 20, No. 2, pages 237 - 246) describes the use of a poly(L-lysine)-g-poly(ethylene glycol) (PLL-g-PEG) to reduce nonspecific adherence of macrophages and dendritic cells on PLL-g-PEG coated glass surfaces and to reduce nonspecific phagocytosis of PLL-g-PEG coated particles. A PLL-g-PEG, modified with short peptides mediating receptor-specific interaction, is also reported.

Mueller et al. (J Biomed Mat Res 2003 66A (1), 55 - 61) describes the decreased protein adsorption to PLL-g-PEG coated poly(lactic-co-glycolic acid) (PLGA) microspheres. The introduction of functional groups on PLGA microspheres via functional PLL-g-PEG is also described.

None of the implants available so far provides optimal results in all aspects crucial for successful implantation. Rough surfaces improve osteo-integration, but the implant/tissue interaction is nonspecific. Surfaces coated for improved resistance to protein and cell adherence also impair attachment of cell types wanted, e.g. osteoblasts involved in the synthesis of new bone substance. In addition, the flexibility to customize these types of implants, for instance with desired bioactive agents, just prior to their use is fairly poor.

It is an object of the present invention to provide an implant comprising a surface showing low nonspecific implant/tissue interaction and providing for the necessary bioactive factors released in a controlled manner allowing a fast healing, which includes preventing acute or chronic inflammatory processes and a quick and mechanically stable osseo-integration.

It is another objective of the present invention to provide a kit for preparing an implant according to the present invention.

Implant as used herein refers to any object which is at least partly integrated in the bone or which is designed to be at least partly integrated in the bone in the course of the healing process. The object comprises tissue-compatible metals or mixtures of such metals (alloys). In addition, the implant can comprise an oxide layer on its surface.

These objects are achieved by an implant according to claim 1 and a kit according to claim 23. Preferred embodiments are the subject-matter of dependent claims.

Surprisingly, it has been found that the combination of quite few "building blocks" provides the flexibility to furnish an implant with bioactive agents without deteriorating integration properties and protein or cell resistance. On the contrary, implant/cell interactions may be driven in the direction wanted. The growing demand for implants with functionalized surfaces providing for a controlled release of bioactive agents tailor-made to the situation of the individual patient is also met by implants according to the present invention. These advantages render implants according to the present invention superior to commercially available implants. As a consequence, this allows the use of implants according to the present invention also in the case of risk patients, e.g. patients suffering from diabetes, bone diseases, vascular and kidney diseases, patients having genetic deficiencies or vitamin D deficiencies. Further groups of risk patients include heavy smokers or patients undergoing or underwent a chemotherapy or radiative therapy. Implants according to the present invention promise also in these cases a good osseo-integration.

The "building blocks" needed to realize an implant according to the present invention are (i) an implant with a roughened surface conferring excellent osseo-integration, (ii) an intermediate layer which provides for the attachment of a top layer by a linker selected from the group consisting of nucleic acids, PNA, peptides, proteins, glycosylated proteins and carbohydrates, and which creates an attractive environment for wanted cells such as osteoblasts via specific peptide sequences (e.g. RGD, KRSR providing attachment sites) and at the same time conferring resistance to nonspecific protein and cell adherence and (iii) a top layer capable of binding to the intermediate layer and providing for the controlled release of bioactive agents. In addition, the top layer can also provide attachment sites for wanted cells.

Surprisingly favorable properties of an implant according to the present invention are the virtually abolished non-specific interaction between the roughened implant surface and its tissue environment while in parallel adhesion and proliferation of osteoblasts are promoted. In other words, interactions at the implant/tissue interface may not only be driven in one suitable direction they are in fact controllable. The different aspects of the present invention are described in more details below.

An implant according to claim 1 has a surface which is at least partially roughened. The surface may be prepared by machining and structuring, sandblasting or other suitable methods (e.g. plasma cleaning) and subsequent chemical treatment, e.g. etching with an acid. Especially, a sandblasted implant is etched with an inorganic acid or a mixture of inorganic acids, preferably hydrofluoric acid, hydrochloric acid, sulphuric acid or a mixture of these acids

The roughened surface is at least partially covered by organic or polymeric compounds forming an intermediate layer. The coverage may also be complete. The thickness of the intermediate layer is much smaller than the dimensions of the macro- and micro-roughness in order to protect the coating from shear stress. Preferably, the intermediate layer forms a monolayer on the roughened implant surface. The intermediate layer has three main functions, first it prevents unwanted non-specific adsorption to the implant surface, second it provides for the attachment of a top layer by a linker and third it can provide attachment sites for osteoblasts, thus creating a osteogenic environment. Nonspecific adsorption may occur in several ways. First, proteins, e.g. from blood, may adsorb to the implant surface, second, cells coming in close contact with the implant surface may adhere to it. Since the insertion of an implant in consequence also interferes with the tissue integrity, it is likely that cells of the immune system are attracted to the implantation site. This, however, often leads to inflammatory processes severely compromising the successful integration of the implant. Third, the implantation site necessarily comes into contact with its environment which facilities the penetration of bacteria. Thus, it is important that the implant surface does not promote the adherence of bacteria. The implants according to the present invention fulfill the need for implants with biologically inert surfaces displaying low nonspecific protein adsorption or nonspecific adherence of phagocytic cells. There are different growth factors such as TGFβ, BMP-2 and PDGF exerting an effect on osteoblast in that they stimulate differentiation and proliferation of these cells. Attachment of osteoblasts is at least partly conferred via integrin receptors binding to the RGD peptide. This motif is found in various extracellular proteins.

The top layer comprises at least one bioactive agent and provides for a controlled release of the bioactive agent.

It may also comprise RGD sequences for specific attachment. The top layer is not necessarily formed as a continuous and complete layer. It may also be made up by carrier structures comprising at least one bioactive agent. The density of such carriers depends on the density of attachment sites provided by the intermediate layer. Preferably, the carriers also comprise a coating in order to prevent nonspecific adsorption of proteins or nonspecific adherence of cells or bacteria to the carriers. If the coating of the carrier structures also comprises short peptide sequences, e.g. RGD or KRSR, sites for specific attachment are provided. This can confer attachment to the intermediate layer. Further, free RGD sequences can also mediate attachment to cells expressing the appropriate receptor. For instance, osteoblasts can bind to RGD sequences through their integrin receptors.

Attachment is conferred by linkers, which comprise either one, two or more parts. Preferably, these linkers comprise two parts, whereby one part is covalently bound to the intermediate layer and the counterpart is covalently bound to the carrier or its coating. The linkers can also be non-covalently bonded to the intermediate layer and the carrier.

The attachment between the intermediate layer and the top layer may comprise covalent bonds, ionic bonds, hydrophobic interaction, hydrogen bonds, electrostatic bonds or a combination thereof.

As a consequence of the rough surface topography, the intermediate and the top layer attached thereto are "buried" in the implant surface. In other words, the dimensions of the surface roughness are greater by several orders than the dimensions of the intermediate and the top layer together. This confers protection against mechanical forces (e.g. shear forces at the time of implantation) to the intermediate and the top layer.

When PLL-g-PEG is used to form the intermediate layer, the bonding between the implant surface and the intermediate layer is electrostatic (action between bodies coming from charges), since the positively charged side chains of the PLL bind to the negatively charged metal surface. In addition, other bonding types are possible, for instance DOPA-PEG binds covalently to the metal surface.

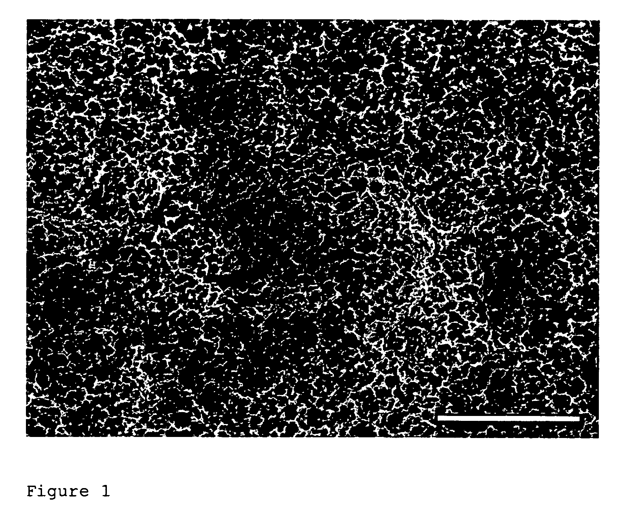

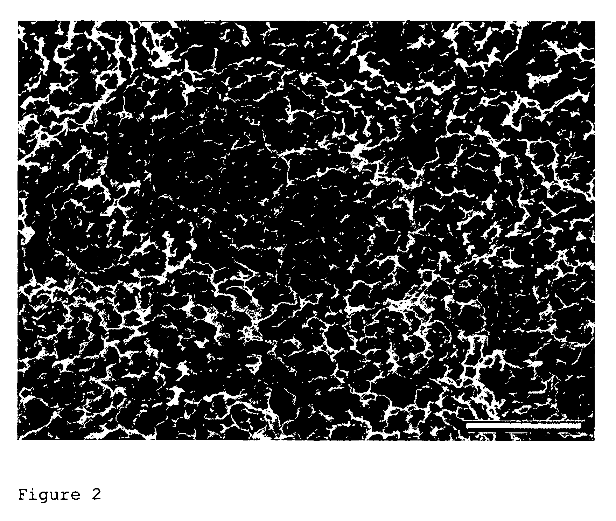

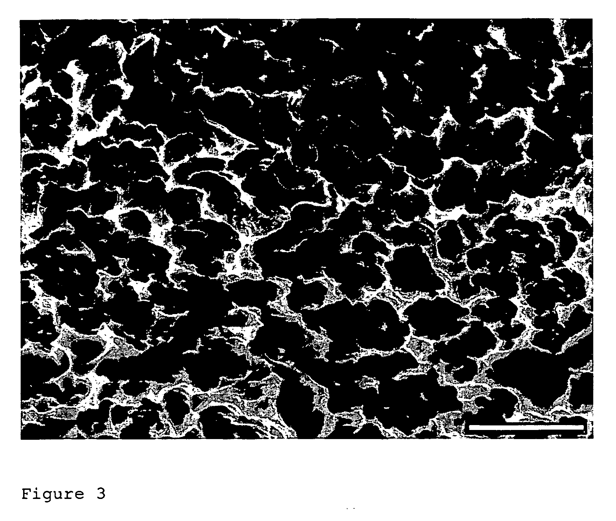

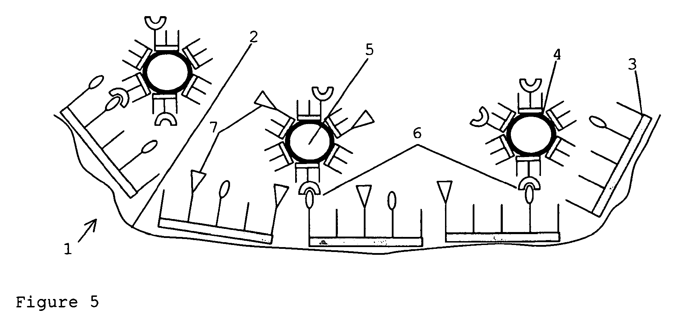

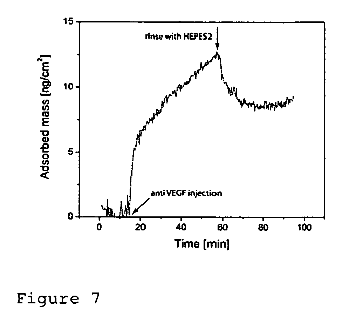

Figures 1 to 5 schematically illustrate the different dimensions of the intermediate and the top layer in relation to the roughness of the implant surface. The figures show an implant (1) having a roughened and possibly hydroxylated surface (2). The implant surface (2) is covered by an intermediate layer (3), attached thereto is a top layer (4) comprising carrier structures (5). The attachment is provided by linkers (6, 6', 7).
Figure 1 shows a section of an implant surface illustrating a macro-roughness. The white bar in the lower right corner illustrates a distance of 50µm;
Figure 2 shows a section of an implant surface illustrating a macro-roughness in greater magnification. The white bar in the lower right corner illustrates a distance of 20µm;
Figure 3 illustrates a micro-roughness superposing the macro-roughness. The white bar in the lower right corner illustrates a distance of 10µm;

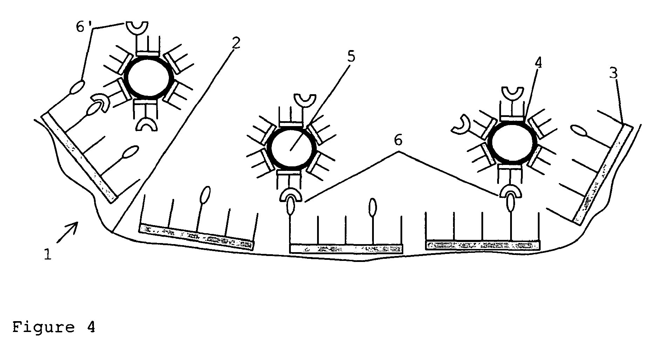

Figure 4 illustrates in more detail the components of the intermediate and the top layer. The brush-like structures covering the implant surface (2) form the intermediate layer (3). Attached to the intermediate layer are carrier structures (5) forming the top layer (4) and comprising at least one bioactive agent. The carrier structures (5) are coated in a similar way as the implant surface. Attachment is conferred via linker structures (6) comprising e.g. peptides binding to heparin. The linker structures are not necessarily all engaged in providing attachment between the intermediate layer and the top layer. There may also be free linker structures (6'). If appropriately chosen, free linkers acquire another important function in the healing process. They provide attachment sites for wanted cells such as osteoblasts thereby specifically facilitating immigration of these cells, e.g. the peptide KRSR. The density of the carriers forming the top layer may vary according to the density of the attachment sites provided by the intermediate layer or according to the amount of the available carriers; and
Figure 5 shows a section similar to that in figure 4. In this embodiment the intermediate (3) and the top layer (4) comprise different linker structures (6) and (7), which are not necessarily engaged in the attachment between the intermediate and the top layer. Some linker structures may specifically provide attachment sites for wanted cell types which immigrate in the course of the healing process.
Figure 6 shows a Western Blot specific for TG-VEGF. Western blots are treated with antibodies against the desired protein, in this case VEGF, to detect it. By knowing the molecular weight of free TG-VEGF, a coupling to a higher molecular weight molecule through can be shown, as only bands containing VEGF are visible. Lanes (from left to right) are:
   1. PLL-*g*-PEG/PEG-Lys incubated with VEGF
   2. PLL-*g*-PEG/PEG incubated with VEGF
   3. PLL-*g*-PEG/PEG-Lys reacted with TG-VEGF; 2µg VEGF
   4. PLL-*g*-PEG/PEG-Lys reacted with TG-VEGF; 1µg VEGF
   5. Supernatant of reaction preparation
   6. Marker
   7. TG-VEFG control; 1µl
   8. TG-VEGF control; 5µl
   9. Control TG-VEGF reacted to PEG, purified
   10. Bulk beads after dissociation of co-polymer
Figure 7 illustrates an OWLS measurement of TG-VEGF coupled to PLL-g-PEG/PEG-Lyspep. The TiO₂-coated waveguide was incubated ex situ with PLL-g-PEG/PEG-Lyspep and coupled with TG-VEGF at 37 °C. Subsequently, the modified waveguide was introduced into the OWLS instrument and after, reaching a stable baseline, antiVEGF antibody was injected.

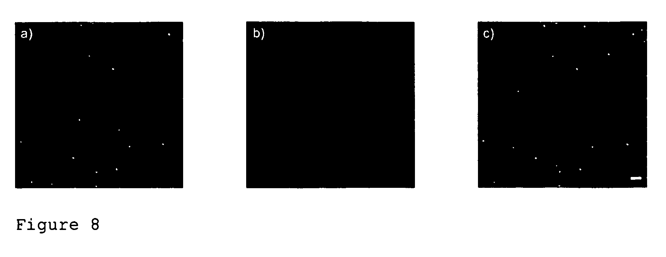

Figure 8 shows confocal microscopy images of loaded and PLL-g-PEG coated liposomes (80 wt% DOPC, 20 wt% DOPS; 100 nm in diameter) adsorbed on a glass slide. a) Liposomes loaded with 20 mM FITC in PBS solution (fluorescently green). b) Liposomes coated with CY3-labeled PLL-g-PEG polymer (fluorescently red). c) Superposition of image a) and b). Correlation coefficient is r=0.89. Scale bar corresponds to 300 µm.

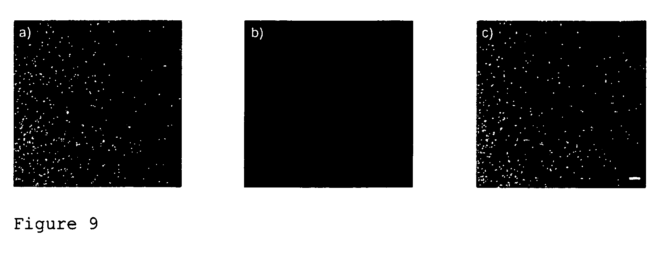

Figure 9 shows confocal microscopy images of fluorescently labeled liposomes (79 wt% DOPC, 20 wt% DOPS, 1 wt% NBD-PC; 100 nm in diameter) that were coated with PLL-g-PEG polymer, adsorbed on a glass slide. a) Liposomes containing 1 wt% fluorescently labeled phospholipids (fluorescently green). b) Liposomes coated with CY3-labeled PLL-g-PEG polymer (fluorescently red). c) Superposition of image a) and b). Correlation coefficient is r=0.91. Scale bar corresponds to 300 µm.

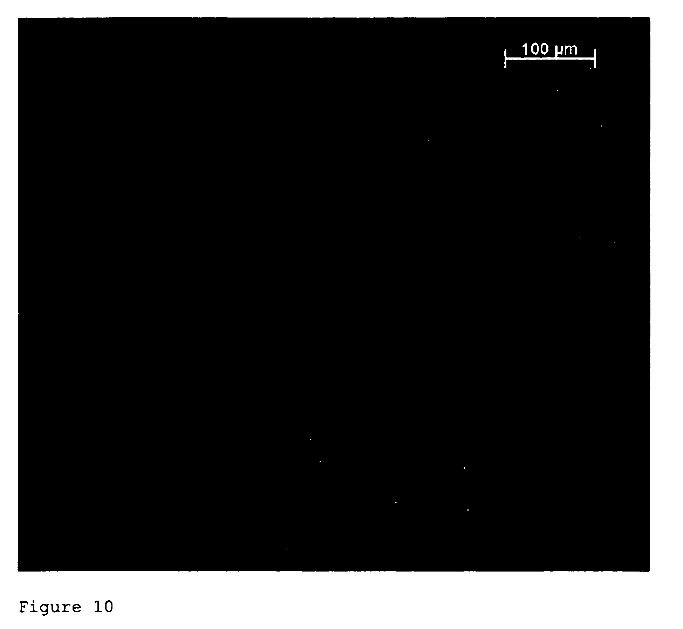

Figure 10 illustrates the coupling of PLL-g-PEG/PEG-TGpep modified PLGA microspheres to a TiO₂ surface via PLL-g-PEG/PEG-Lyspep chemistry. On a MAPL-patterned surface PLL-g-PEG/PEG-FITC (fluorescein isothyocianate-labeled (green) PLL-g-PEG polymer) was deposited. After photoresist removal, PLL-g-PEG/PEG-Lyspep was deposited in the "empty" area (developed region) leading to a surface with a color contrast green vs. black. Model drug delivery carriers were subsequently coated with a mixture of PLL-g-PEG/PEG-TG and PLLg-PEG/PEG-CY3 (Carbocyanin-3-labeled (red) PLL-g-PEG polymer). Microspheres were washed and resuspended in a reaction mixture containing the transglutaminase and then transferred to the MAPL surface. Since coupling is specific, red fluorescent areas were visible by fluorescence microscopy in the PLL-g-PEG/PEG-Lyspep coated area surrounding green fluorescence in the non-binding coated spots (in a black and white image, the dark-grey squares correspond to PLL-g-PEG/PEG-FITC whereas light-grey areas refer to PLL-g-PEG/PEG-CY3).

An implant according to claim 1 has an improved biocompatibility in terms of reduced nonspecific protein adsorption. Also, nonspecific cell adherence or the adherence of bacteria to the implant surface is greatly diminished ( Harris, L.G., et al., Staphylococcus aureus adhesion to titanium oxide surfaces coated with non-functionalized and peptide-functionalized poly(L-lysine)-grafted-poly(ethylene glycol) copolymers. Biomaterials, 2004. 25(18): p. 4135-4148). On the other hand, the bioactive agent(s) comprised in the top layer allows the triggering of directed interactions of the implant surface with its tissue environment. For instance, implants which are inserted into the bone (e.g. the jaw in the case of dental implants) ideally promote the immigration and the attachment of osteoblasts, thereby achieving an accelerated, stable integration of the implant and a fast healing. These directed interactions may be tailored to the needs of the individual patient.

The implant is made of metal or a composition of different metals (alloy). The implant can also comprise a metal oxide layer on its surface.

In a preferred embodiment the implant has a surface which is at least partially roughened and at least partially hydroxylated. The roughened and the hydroxylated surface area is not necessarily identical, although this is usually the case. Briefly, the roughening is achieved for instance by sandblasting and the hydroxylation is achieved for instance by etching or plasma-treatment.

The surface may be prepared by machining and structuring, anodic oxidation, sandblasting or other suitable methods and subsequent chemical treatment, e.g. etching with an acid, exposition to UV-radiation or by using an electrolytic treatment, or by a combination of such processes. Especially, a sandblasted implant is etched with an inorganic acid or a mixture of inorganic acids, preferably hydrofluoric acid, hydrochloric acid, sulphuric acid, nitric acid or a mixture of these acids, or alternatively, the surface is activated by a mixture of hydrochloric acid, hydrogen peroxide and water in the ratio 1:1:5 based on the weight.

In another preferred embodiment, the employed metals are titanium, zirconium, niobium, tantalum, or hafnium, or compositions (alloys) of these metals.

In a further preferred embodiment, the rough surface comprises a dual roughness. First, there is a macro-roughness with pit sizes greater than 10µm, preferably more than 20µm, and second, a micro-roughness with pit sizes of 4µm or less, preferably 2µm or less. Methods to obtain such surfaces are known in the art. EP 0 388 576 discloses a method wherein the above described surface is obtained by sandblasting and subsequent etching with an acid. WO 00/44305 discloses methods for obtaining metal implants, preferably based on titanium or titanium alloys, with a roughened and hydroxylated surface. In addition, it is disclosed how the hydroxylated state may be preserved until the implant is used. EP 0 388 576 and WO 00/44305 are incorporated by reference herein.

In a preferred embodiment, the intermediate layer is a functionalized graft-copolymer. Such graft-copolymers are particularly suited to form the intermediate layer, since they are easily functionalized. Functionalizing means that specific moieties are attached to the graft-copolymer. The attachment is preferably covalent. It is known to one skilled in the art how to achieve such functionalization. The specific moieties may be selected from peptides, proteins, glycosylated proteins (including growth factors), carbohydrates or other suitable compounds or combinations thereof.

In a further preferred embodiment, the graft copolymer is poly(L-lysine)-graft-poly(ethylene glycol) (PLL-g-PEG). PLL-g-PEG has a number of advantages. It may be easily functionalized and it does readily adsorb to implant surfaces, especially to negatively charged metal or metal oxide surfaces. Intermediate layers comprising PLL-g-PEG show a high resistance to nonspecific protein adsorption and nonspecific cell adherence, thus greatly reducing unwanted inflammatory processes.(Tosatti, S., et al., Peptide functionalized poly(L-lysine)-g-poly(ethylene glycol) on titanium: resistance to protein adsorption in full heparinized human blood plasma. Biomaterials, 2003. 24(27): p. 4949-4958; Tosatti, S., Functionalized Titanium Surfaces for Biomedical Applications: Physico-chemical Characterization and Biological in vitro Evaluation, in Departement of Materials. 2003, ETH: Zurich. p. 386). One skilled in the art easily recognizes that there are many variations, too numerous to be mentioned, of the PLL-g-PEG which can be used, e.g. the poly-lysine chains can comprise additional compounds such as other amino acids. In addition, DOPA-PEGs can be employed, where DOPA means L-3,4-dihydroxyphenylalanine, an amino acid found in mussel adhesive proteins. DOPA-PEGs bind covalently to titanium surfaces. Further examples are poly-phosphonate-copolymers and pegylated SAMs (Self-Assembled Monolayers).

The top layer is bound to the intermediate layer via linkers. As linkers may serve the above mentioned moieties used to functionalize graft copolymers. The binding is then accomplished with a counterpart comprised in the top layer. Thus, the linker comprises usually two parts.

In another preferred embodiment the linkers are selected from the peptide KRSR and FHRIKKA. These peptides are known to bind to heparin. Therefore, heparin is the counterpart which must be present in the top layer if the intermediate layer comprises at least one of the above mentioned peptides. It is not decisive if the peptides are comprised in the intermediate layer and the heparin, as the counterpart, is comprised in the top layer. The arrangement may also be vice versa. Thus, the linker may also comprise more than two parts. For instance, the intermediate layer and the top layer may both comprise the peptide KRSR or a multimer thereof (e.g. (KRSR)₃) which both bind to heparin or heparansulfate. Thus, the attachment is achieved by adding heparin or heparansulfate.

A further example how to achieve the linkage between the intermediate layer and the top layer are the peptides KRSR or (KRSR)₃ binding to a short heparin sequence (sometimes also referred to as "chopped heparin") which can bind to another KRSR or (KRSR)₃ peptide. The chopped heparin is obtained by enzymatic digestion of heparin.

The sulfated tetrapeptide SY(SO₃)DY(SO₃) is a short peptide mimicking heparin and heparansulfate. The tetrapeptide has been obtained by a combinatiorial approach (Discovery of a sulfated tetrapeptide that binds to vascular endothelial growth factor, Heather D. Maynard, Jeffrey A. Hubbell, Acta Biomaterialia, 2005). A linkage can be achieved via the binding of SY(SO₃)DY(SO₃) or a mulitmer therof to KRSR or a multimer thereof.

Peptide nucleic acids (PNA) may also be employed as linker (e.g. the PNA (NH₂-GCR-AGT CCA TTG CTG AAG) binding to the complementary PNA (NH₂-GCR-CTT CAG CAA TGG ACT). PNA were originally conceived and designed as sequence specific DNA binding reagents targeting the DNA major groove in analogy to triplex forming oligonucleotides. However, instead of the sugar-phosphate backbone of oligonucleotides PNA was designed with a pseudopeptide backbone. It also quickly became clear that triplexes formed between one homopurine DNA (or RNA) strand and two sequence complementary PNA strands are extraordinarily stable.

Another example for linking the intermediate layer with the top layer is the use of lysine-containing (Lyspep) cleavable (e.g. N-acetyl-F**K**GGG**PQGIWGQ**ERCG-amide, mmp-2 cleavable) or non-cleavable peptides (e.g. N-acetyl-F**K**GGGDQGIAGFERCG-amide) that covalently bind to glutamine-containing peptides (TGpep, e.g. COOH-NQE**Q**VSPLERC-amide). The formation of a covalent bond between the free amine group of lysine and the gamma-carboxamid group of glutamine is catalyzed by a transglutaminase (e.g. Factor XIIIa). These bonds are highly resistant to proteolytic degradation.

In a further preferred embodiment the top layer comprises carriers. Such carriers may be lipid vesicles, polyelectrolyte stabilized vesicles, micro-spheres or nano-particles. Micro-spheres can be small spherical or small extended particles. They can be solid or hollow and are made of a variety of materials such as polymers, ceramics, minerals, etc. The carriers comprise at least one bioactive agent, which is subsequently delivered to the local environment of the implant. Depending on the composition of the carrier the release of the bioactive agent can be controlled.

In a preferred embodiment the carrier comprises poly(lactic acid) (PLA) or poly(lactic-co-glycolide acid) (PLGA). Micro-spheres prepared with these polymers can contain bioactive agents. In addition, such micro-spheres are highly biocompatible and biodegradable. The rate of their degradation, and thus the release of a bioactive agent, can be controlled by modulating their composition. To prevent nonspecific phagocytosis it is beneficial if the micro-spheres are coated in a similar fashion to the implant surface. At the same time the coating allows to implement the counterpart to a linker on the intermediate layer mentioned above.

Bioactive agents which can be employed are manifold. For instance the agents may be selected from systemic hormones, cytokines, growth factors, anti-inflammatory drugs or the like. One improvement of the present invention is the possibility to adjust the bioactive agents comprised in the carriers to the situation of the patient.

In a preferred embodiment, the bioactive agents are selected from the group of systemic hormones (e.g. growth hormone (GH), insulin-like growth factor 1 (IGF-1), thyroid hormone, vitamin D, estrogens, androgens, selective estrogen receptor modifiers (SERMs), glucocorticoids and parathyroid hormones (PTH)), growth factors and cytokines (e.g. transforming growth factors β (TGF-β), bone morphogenetic proteins (BMPs), fibroblast growth factors, parathyroid hormone related peptides (PTHrP), prostaglandins (PGs), platelet-derived growth factors (PDGFs), vascular endothelial growth factors (VEGF), osteoprotegerin, substances extracted from the dental enamel matrix known as Enamel Matrix Derivatives (EMD), calcium, biphosphat, fluorides, anti-inflammatory and any mixtures thereof.

In a preferred embodiment, the bioactive agent is selected from the group of the parathyroid hormones, VEGFs and the bone morphogenetic proteins.

In a further preferred embodiment the bioactive agent is selected from the bone morphogenetic proteins (BMPs), particularly preferred are BMP-2 and BMP-7.

In a further preferred embodiment the bioactive agent is selected from the parathyroid hormones.

In another preferred embodiment, the bioactive agent is selected from the substances extracted from the dental enamel matrix known as Enamel Matrix Derivatives (EMD) such as Amelogenin, Amelin or Enamelin.

All these bioactive agents play, among other factors, a role in the formation and the morphogenesis of bone tissue.

Kits also fall within the scope of the present invention. These kits are useful for in situ adsorption of the intermediate layer on the roughened implant surface and subsequent forming of the top layer on the coated implant prior to implantation surgery.

A kit comprises at least three individual containers in which the components (i) an implant having at least partially a roughened surface, (ii) a component for forming the intermediate layer comprising a linker selected from the group consisting of nucleic acids, PNA, peptides, proteins, glycosylated proteins and carbohydrates and (iii) a component for forming the top layer with at least one bioactive agent, are stored.

The kit may also comprise more than three containers with different components (ii) for the intermediate layer and/or different components (iii) for the top layer with at least one bioactive agent. The components (ii) may be mixed before the implant is soaked therein. The same applies to the components (iii). Thus, a kit comprising three individual containers with an implant, the components (ii) and (iii) illustrates a preferred kit. The kits may also comprise more than three containers to provide the necessary flexibility in selecting the appropriate components. For instance, it is possible to employ two different components (ii) in a desired ratio each component (ii) comprising a different linker. The different linkers allow the use of two components (iii) comprising different bioactive agents. It is also possible that one of the linkers employed does not provide attachment sites for carriers with bioactive agents but attachment sites for specific cell types favoring the attachment and the in-growth of such cells.

If more than one component (ii) and more than one component (iii) are used, the different components (ii) and the different components (iii) are premixed in separate containers, yielding two premixes, one comprising the different components (ii) and the other comprising the different components (iii). Afterwards, the implant can be soaked in the premix of the different components (ii) and subsequently in the premix of the different components (iii). In such a way the implant may be customized to the needs of the patient.

It is also possible to store the components (ii) and (iii) in solid form instead of a solution. This has the advantage of an even better stability of the different components (ii) and (iii) which consequently leads to a longer shelf live of the kit.

A dentist or physician would soak the implant first in a solution comprising the component (ii) for forming the intermediate layer and then in a solution comprising the component (iii) for forming the top layer with at least one bioactive agent. The procedure can be done in a very short time, taking some seconds up to a few minutes.

In a further embodiment, the kit comprises an implant having at least partially a roughened and partially hydroxylated surface.

In a preferred embodiment, the components (ii) for forming the intermediate layer and (iii) for forming the top layer are individually stored in containers.

Since the components of the kit are individually stored in containers under sterile conditions and opened just prior to their use, utmost sterility is ensured.

Implants as described herein may also be used as dental implants, e.g. in the form of pins to be screwed into the jaw for constructing artificial teeth.

In another preferred embodiment individually stored components (ii) and (iii) are used to coat an implant having at least partially a roughened or at least partially a roughened and partially hydroxylated surface.

### Example 1: selective cell adhesive surface with drug eluting capabilities

### A. Manufacturing of rough metallic substrate:

The rough SLA implant surface is produced by grit blasting process with corundum particles (ø250µm, pressure 5 bar) that leads to a macro-roughness on the titanium surface. This is followed by a strong acid-etching bath with a mixture of HCl/H₂SO₄ at elevated temperature for several minutes, as described in EP0388576. This produces the fine 2-4 µm micro-pits superimposed on the rough-blasted surface. The surface is not microporous and therefore provides no enclosed volumes to reduce vulnerability to bacteria.

The chemical composition of the SLA structure was found to be titanium oxide (TiO₂) using X-ray photoelectron spectroscopy. This method analyses the first few atomic layers of the surface, and thus the chemical composition of the material which is in direct contact and interacts with tissue fluids and cells.

### B. Synthesis of Poly(L-lysine)-g-poly(ethylene glycol):

Synthesis of a polymer with assembling properties, capable to minimize non-specific interaction while carrying cell-interaction selective groups and or biologically-like interacting groups and specific functionalization (biological and/or linkable) and its deposition on a metallic substrate as described in A:

The five functionalized PLL-*g*-PEG/PEG-peptide polymers (RGD, KRSR, Lyspep (cleavable and non-cleavable) and TGpep) were synthesized using peptides with the following amino acid sequences: N-acetyl-GCRGYGRGDSPG-amide, N-acetyl-GCRGYGKRSRG-amide, N-acetyl-F**K**GGG**PQGIWGQ**ERCG-amide, N-acetyl-F**K**GGGDQGIAGFERCG-amide, COOH-NQE**Q**VSPLERC-amide. The synthesis was performed in a Pioneer peptide synthesizer using standard Fmoc techniques. The HPLC-purified samples were analyzed using MALDI-ToF. Aminoacids were purchased from Novabiochem, Switzerland. The synthesis of the PLL-*g*-PEG/PEG-peptide was performed as described in detail elsewhere (Huang, N.P., et al., Poly(L-lysine)-g-poly(ethylene glycol) layers on metal oxide surfaces: Surface-analytical characterization and resistance to serum and fibrinogen adsorption. Langmuir, 2001. 17(2): p. 489-498; VandeVondele, S., J. Voros, and J.A. Hubbell, RGD-Grafted poly-l-lysine-graft-(polyethylene glycol) copolymers block non-specific protein adsorption while promoting cell adhesion. Biotechnology and Bioengineering, 2003. 82(7): p. 784-790). In brief, poly(L-lysine) (MW 15-30 kDa from Sigma, USA) was reacted with methoxy-PEG-SPA (2 kDa) and NHS-PEG-VS (3.4 kDa, both activated polymers from Shearwater, USA) in a ratio of 3.5 (number of lysine monomer units) to 0.95 (number of mPEG-SPA chains) to 0.05 (number of NHS-PEG-VS chains). The reaction was allowed to proceed in a pH 8.4 buffer for 30 min, then a cysteine containing peptide was added in a 2-fold excess when compared with the number of NHS-PEG-VS molecules. The reaction was allowed to continue overnight and was followed by the addition of 2-mercaptoethanol to quench the remaining free vinyl sulfones. The reaction among the peptides, the PLL and the PEG derivatives resulted in a clear liquid. The salts and 2-mercaptoethanol were dialyzed away (DI water, 3 days) and the samples were subsequently lyophilized. The polymers were characterized as described by Tosatti, et *al.* (Tosatti, S., et al., Peptide functionalized poly(L-lysine)-g-poly(ethylene glycol) on titanium: resistance to protein adsorption in full heparinized human blood plasma. Biomaterials, 2003. 24(27): p. 4949-4958): The grafting ratio, g, which corresponds to the ratio of the number of lysine monomer units to the number of PEG side chains, was determined by NMR. Best results in terms of protein resistance are achieved with 3 < *g* < 5. The degree of functionalization was determined with the Tyrosine (Y) from the used amino acid sequences with its specific peak for quantification.

Coating of the surface: 0.5 mg/ml of each polymer (PLL-g-PEG/PEG-RGD and PLL-g-PEG/PEG-KRSR) were dissolved in HEPES buffer, pH 7.4, filter sterilized (Millex-GW, Millipore, Switzerland), aliquoted and stored at -20 °C. Prior to surface modification, the titanium samples were ultra-sonicated for 5 minutes in 2-propanol (UVASOL, Merck, Dietikon, Switzerland) to remove adventitious macroscopic contamination and dried under nitrogen. After cleaning and sterilizing in an oxygen plasma (Harrick Plasma Cleaner/Sterilizer PDC-32G, Ossining, NY, USA) for 3 minutes, the surfaces were immediately placed in cell culture wells of 24-well plates. The polymer solutions were pipetted onto the sterile surfaces so that the surfaces were just covered by liquid. The samples were then gently shaken for 15 minutes, and washed three times with sterile HEPES, one with water, dried under nitrogen and stored for further usage. *C*. *Loading of a drug delivery system with a biologically active molecules and coating with a similar polymer as described in B*

Bioactive agents can be encapsulated in nanospheres, PLGA microspheres, liposomes, or by other methods for the purpose of slowing down their release or of protecting them from unwanted modifications (Bodmeier R, McGinity JW. Solvent selection in the preparation of poly(DL-lactide) microspheres prepared by the solvent evaporation method. Int J Pharm. 1988,43:179-186).

Lyophilized rhBMP-2 (Recombinant human bone morphogenetic protein) and 50:50 PLGA poly(lactic-co-glycolic acid) porous microspheres were used (Schrier JA, DeLuca PP. Recombinant human bone morphogenetic protein-2 binding and incorporation in PLGA microsphere delivery systems. Pharm Dev Technol. 1999;4:611-621). Lyophilized protein was reconstituted with water for injection; the resulting solution was concentrated approximately 10-fold by ultrafiltration on a membrane. Protein solution was aseptically filtered with 0.2 mm filters and stored at 4°C.

The PLGA microspheres were suspended in a protein solution and allowed to equilibrate for 24 hours at room temperature (RT) before recovery by filtration on a 0.45 µm low-protein-binding filter. Wet microspheres were lyophilized via (1) freezing at -45°C for 6 hours, (2) primary drying at 15°C, 150 mTorr for 12 hours, and (3) secondary drying at 25°C, full vacuum, for 6 hours. Subsequent quantification of "free," "bound," and "total" rhBMP-2 associated with the microspheres was carried out by using a simple protein mass balance and the assumption that free protein concentration in the PLGA microspheres was equivalent to that in the separated rhBMP-2 solution following the filtration step. Free protein referred to that present on the surface and within the microspheres' pores of the PLGA matrix, whereas bound protein referred to that physically adsorbed. Loaded microspheres were assessed by scanning electron microscopy and for protein load and in vitro release.

Coating of the beads: Microspheres were dispersed in filtered HEPES buffer at a concentration of 1 mg/mL (PLGA microspheres). The dispersions were mixed at equal volumes with the solution containing both PLL-*g*-PEG-peptide polymers (1 mg/mL in filtered HEPES buffer) and incubated under gentle mixing for 15 min at room temperature. The coated microspheres were centrifuged (5 min, 7,400 x g, Eppendorf, Centrifuge 5417R, Eppendorf-Netheler-Hinz GmbH, Germany) and redispersed in half of the previous volume in filtered HEPES buffer by slightly vortexing.

### D. Linkage of the drug delivery system as obtained from C to a surface as obtained in B

Surfaces obtained on B are incubated in 1mg/ml Heparin solution (Buffer HEPES, pH=7.4) for 30 min and rinsed three times with HEPES. Afterward the samples were soaked in the solution containing the functionalized drug delivery beads as obtained in C for 30 min, and washed three times with sterile HEPES, one time with water, dried under nitrogen and stored for further usage. Drying under nitrogen may be omitted, the implants may also be directly stored while still wet.

### E. Linkage of a bioactive agent to a surface as obtained in B

Frozen samples of PLL-g-PEG/PEG-Lyspep powders were warmed up to room temperature, dissolved in a salt buffer solution (denoted hereafter as TBS) containing 10 mM Tris and 150 mM NaCl at pH 7.4 (to reach a final concentration of 0.5 mg/ml), filter sterilized (0.22 µm filter, Milian, Basel, CH) and used to coat the surfaces. Titanium substrates were ultra-sonicated for 5 minutes in 2-propanol (UVASOL, Merck, Dietikon, Switzerland) to remove adventitious macroscopic contamination and dried under nitrogen. After cleaning and sterilizing in an oxygen plasma (Harrick Plasma Cleaner/Sterilizer PDC-32G, Ossining, NY, USA) for 3 minutes, samples were coated with PLL-g-PEG/PEG-Lyspep polymer solutions. Substrates were washed twice with TBS after 30 min, and following reaction mixture, containing 5 µg of a growth factor carrying a TG-sequence (e.g. TG-VEGF), 10 µl of 1M CaC12 (Sigma), 12 µl Thrombin (human Thrombin, Sigma) activated Factor XIII and 174 µl TBS, was added to covalently link transglutaminase substrate site (TG)-modified VEGF to the polymer. The substrates were incubated at 37°C for 45 min and subsequently washed twice with TBS.

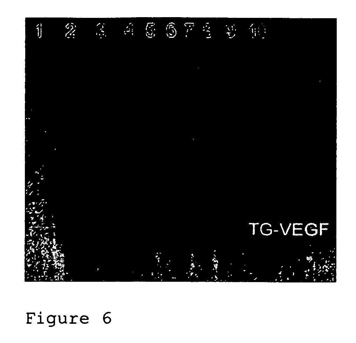

Coupling between TG-VEGF and Lyspep was shown by Western Blot (see Figure 6). Western Blot towards VEGF protein showed a weight shift towards high molecular weights.

Successful linking of TG-VEGF to titanium surfaces coated with PLL-g-PEG/PEG-Lyspep was demonstrated by optical waveguide lightmode spectroscopy (OWLS; Voros J, Ramsden JJ, Csucs G, Szendro I, De Paul SM, Textor M, et al. Optical grating coupler biosensors. Biomaterials. 2002 Sep;23(17):3699-710). A TiO₂-coated waveguide was incubated ex situ with PLL-g-PEG/PEG-Lyspep and coupled with TG-VEGF as described above. Subsequently, the modified waveguide was introduced into the OWLS instrument and after, reaching a stable baseline, antiVEGF antibody (Santa Cruz) was injected and a weight difference of ca. 9 ng/cm² could be measured, indicating the presence of VEGF on the surface (see Figure 7). The negative experiment, consisting of incubating PLL-g-PEG first with TG-VEGF and then with anti-VEGF, showed that the interaction of the anti-VEGF was indeed specific and also that the presence of TG-VEGF was due to the coupling reaction and not to unspecific adsorption.

### F. Production of liposomes

The following two types of phospholipids were used (all purchased from Avanti Polar Lipids Inc.):DOPS (dioleoyl phosphatidyl serine) and DOPC (dioleoyl phosphatidyl choline). Phospholipids were dissolved seperately in chloroform (25 mg/ml) and mixed in a ratio of 0-50 wt % DOPS in DOPC. Solvent was evaporated using a nitrogen stream for 30 min and rehydrated with a water-based buffer (e.g. phosphate buffer saline PBS) to reach a final concentration of 0.5 mg/ml. The obtained solution was vortexed and subsequently filter extruded with a Lipofast extruder (Avestin Inc., Ottawa,Canada) following established protocols (Rossetti FF, et al. Formation of supported phospholipid bilayers on titanium oxide surfaces. Biophysical Journal. 2005 Jan; 88(1):7A-A.). Unilamellar liposomes were characterized by dynamic light scattering technique (DLS; Zetasizer 3000 HSA, Malvern Instruments) to have a mean diameter of 100 nm ± 30 nm.

An alternative approach for a scaled-up production of liposomes (with defined sizes between 30 nm - 100 nm) is the use of repetitive (5-10x) filter extrusion of the above described reaction mixture using a Lipex^{™} extruder (Lipex biomembrane; Zeisberger SM, Odermatt B, Marty C, Zehnder-Fjallman AHM, Ballmer-Hofer K, Schwendener RA. Clodronate-liposome-mediated depletion of tumour-associated macrophages: a new and highly effective antiangiogenic therapy approach. British Journal of Cancer. 2006 Aug;95(3):272-81). Liposomes were characterized by DLS technique to have a mean diameter of 30 nm - 100 nm (± 30%).

### G. Loading of liposomes as described in F

Phospholipids were dissolved, mixed, dried and rehydrated with PBS as described above except that the PBS solution was supplemented with 20 mM FITC (fluorescein-5-thiosemicarbazide; excitation wavelength 495; Molecular Probes) that was used for loading the liposomes. Loading of liposomes was shown through confocal microscopy technique (see Figure 8).

### H. Coating of liposomes as obtained in F or G with PLL-g-PEG polymer

Freshly prepared liposomes were incubated with 0.05-1.5 mg/ml of PLL-g-PEG polymer. Coated liposomes were dialyzed against PBS to remove any free PLL-g-PEG polymer for 48 hours at room temperature (25 °C). PBS was changed twice per day. Coated liposomes were controlled with DLS.

An alternativ approach for the removal of free PLL-g-PEG (without dialysis) is the use of negatively charged microspheres (e.g. COOH-functionalized PLGA microspheres) that were added to the solution (containing PLL-g-PEG and liposomes. Free PLL-g-PEG adsorbed to the microspheres and these were separated by centrifugation technique. Coated liposomes were controlled with DLS.

To prove successful PLL-g-PEG coating of liposomes, fluorescently green labeled liposomes were used. These liposomes were produced using 1 wt% of fluorescently green labeled nitrobenzoxadiazole-phosphatidyl choline (NBD-PC; excitation wavelength 488 nm; 2mg/ml chloroform), 79 wt% of DOPS and 20 wt% of DOPC following the protocol as described in F. Liposomes were coated with fluorescently red labeled PLL-g-PEG polymer (PLL-g-PEG/PEG-Carbocyanin-3; excitation wavelength 488 nm). Liposomes were characterized with DLS which revealed that all free PLL-g-PEG was removed. A superposition of the confocal microscopy images of liposomes and PLL-g-PEG showed a correlation coefficient r=0.91 (as modeled with Matlab software) indicating the coating of the liposomes.

### I. Linkage of drug delivery carriers to a surface as obtained in B

The protocol for coupling drug delivery carriers to PLL-g-PEG/PEG-Lys is the same as for a TG-modified bioactive agent (as described above in E in the example with TG-VEGF) except that the coupling partner for PLL-g-PEG/PEG-Lyspep is now PLL-g-PEG/PEG-TGpep that is used to coat for example poly(lactic-co-glycolic acid) (PLGA) beads or liposomes (as described above in F). The two coupling partners can also be used vice versa, i.e. coating of carriers with PLL-g-PEG/PEG-Lys and linking to the surface via PLL-g-PEG/PEG-TGpep.

To prove the coupling of the drug delivery carriers to the surface (i.d. the intermediate layer), molecular assembly patterning by lift-off (MAPL; Falconnet D, Csucs G, Grandin HM, Textor M. Surface engineering approaches to micropattern surfaces for cell-based assays. Biomaterials. 2006 Jun;27(16):3044-63) technique was used (see Figure 10). On a MAPL-patterned surface PLL-g-PEG/PEG-FITC (fluorescein isothyocianate-labeled (green) PLL-g-PEG polymer) was deposited. After photoresist removal, PLL-g-PEG/PEG-Lyspep was deposited in the "empty" area (developed region) leading to a surface with a color contrast green vs. black. Model drug delivery carriers were subsequently coated with a mixture of PLL-g-PEG/PEG-TG and PLLg-PEG/PEG-CY3 (Carbocyanin-3-labeled (red) PLL-g-PEG polymer). Microspheres were washed and resuspended in a reaction mixture containing the transglutaminase and then transferred to the MAPL surface. Since coupling is specific, red fluorescent areas were visible by fluorescence microscopy in the PLL-g-PEG/PEG-Lyspep coated area surrounding green fluorescence in the non-binding coated spots.

## Claims

1. A metal implant having at least partially a roughened surface, which is at least partially covered by an organic or polymeric intermediate layer and attached thereto a top layer with at least one bioactive agent and said top layer providing for a controlled release of the bioactive agent (s), wherein the top layer is attached to the intermediate layer by a linker selected from the group consisting of nucleic acids, PNA, peptides, proteins, glycosylated proteins and carbohydrates.

2. The implant according to claim 1 wherein the surface is at least partially hydroxylated.

3. The implant of any of the preceding claims, wherein the metal implant comprises titanium, zirconium, niobium, tantalum, hafnium or compositions thereof.

4. The implant of any of the preceding claims, wherein the rough surface comprises a macro-roughness with a pit size greater than 10µm.

5. The implant of claim 4, wherein the rough surface comprises the macro-roughness with a pit size greater than 20 µm.

6. The implant of any of the preceding claims, wherein the rough surface comprises a micro-roughness with a pit size of 4µm or less.

7. The implant of claim 6, wherein the rough surface comprises the micro-roughness with a pit size of 2 µm or less.

8. The implant of any of the preceding claims, wherein the implant is completely covered by an organic or polymeric intermediate layer.

9. The implant of any of the preceding claims; wherein the intermediate layer is a graft copolymer.

10. The implant of claim 5, wherein the intermediate layer is DOPA-PEG.

11. The implant of claim 9, wherein the graft copolymer is poly(L-lysine)-graft-poly(ethylen-glycol).

12. The implant of any of the preceding claims, wherein the top layer is non-covalently bound to the intermediate layer.

13. The implant of claim 1 to 11, wherein the top layer is covalently bound to the intermediate layer.

14. The implant of of any of the preceding claims, wherein the linker is selected from the peptides RGD, KRSR, (KRSR)₃ and FHRIKKA, SY(SO₃)DY(SO₃), heparin and chopped heparin.

15. The implant of any of the preceding claims, wherein the linker is selected from the group consisting of nucleic acids and PNA.

16. The implant of any of the preceding claims, wherein the top layer comprises at least one carrier which is selected from the group consisting of lipid vesicles, polyelectrolyte stabilized vesicles, micro-spheres or nano-particles.

17. The implant of claim 16, wherein the carrier comprises poly(lactic acid) or a poly(lactic-co-glycolide acid) micro-spheres.

18. The implant of any of the preceding claims, wherein the bioactive agent is selected from the group of systemic hormones; cytokines, growth factors, enamel matrix derivatives (EMD) and antiinflammatory drugs.

19. The implant of claim 18, wherein the bioactive agent is selected from the group of parathyroid hormones, VEGF and the bone morphogenetic proteins.

20. The implant of claim 19, wherein the bioactive agent is selected from the bone morphogenetic proteins.

21. The implant of claim 19, wherein the bioactive agent is selected from the parathyroid hormones.

22. The implant of claim 18, wherein the bioactive agent is selected from the enamel matrix derivatives (EMD).

23. A kit for preparing the implant of claim 1 to 22 comprising (i) an individually stored implant having at least partially a roughened surface, (ii) two individually stored components for forming an intermediate layer comprising different linkers selected from the group consisting of nucleic acids, PNA, peptides, proteins, glycosylated proteins and carbohydrates and (iii) at least one individually stored component comprising carriers with at least one bioactive agent.

24. A kit of claim 23 wherein the individually stored implant (i) has at least partially a hydroxylated surface.

25. The kit of claim 23, wherein the components (ii) and (iii) are individually stored in containers.

26. Implant of any of claims 1 to 23, wherein the implant is a dental implant.

27. The use of component (ii), comprising at least one individually stored component for forming an intermediate layer, and of component (iii), comprising at least one individually stores component comprising carriers with at least one bioactive agent, to coat an implant having at least partially a roughened surface.

28. Implant according to claim 27, wherein the implant has at least partially a hydroxylated surface.

## Patentansprüche

1. Metallimplantat, welches mindestens teilweise eine aufgeraute Oberfläche aufweist, die mindestens teilweise von einer organischen oder polymeren Zwischenschicht bedeckt ist, und darauf angebracht eine obere Schicht mit mindestens einer bioaktiven Substanz, und besagte obere Schicht eine kontrollierte Abgabe der bioaktiven Substanz(en) vorsieht, wobei die obere Schicht durch einen Linker, ausgewählt aus der Gruppe bestehend aus Nukleinsäuren, PNS, Peptiden, Proteinen, glykosylierten Proteinen und Karbohydraten, an die Zwischenschicht angebracht ist.

2. Implantat aus Anspruch 1, wobei die Oberfläche mindestens teilweise hydroxyliert ist.

3. Implantat aus einem der vorangehenden Ansprüche, wobei das Metallimplantat Titan, Zirkon, Niob, Tantal, Hafnium oder Zusammensetzungen davon umfasst.

4. Implantat aus einem der vorangehenden Ansprüche, wobei die raue Oberfläche eine Makrorauheit mit einer Kratergrösse grösser als 10 µm umfasst.

5. Implantat aus Anspruch 4, wobei die raue Oberfläche die Makrorauheit mit einer Kratergrösse grösser als 20 µm umfasst.

6. Implantat aus einem der vorangehenden Ansprüche, wobei die raue Oberfläche eine Mikrorauheit mit einer Kratergrösse von 4 µm oder weniger umfasst.

7. Implantat aus Anspruch 6, wobei die raue Oberfläche die Mikrorauheit mit einer Kratergrösse von 2 µm oder weniger umfasst.

8. Implantat aus einem der vorangehenden Ansprüche, wobei das Implantat vollständig durch eine organische oder polymere Zwischenschicht bedeckt ist.

9. Implantat aus einem der vorangehenden Ansprüche, wobei die Zwischenschicht ein Pfropfcopolymer ist.

10. Implantat aus Anspruch 6, wobei die Zwischenschicht DOPA-PEG ist.

11. Implantat aus Anspruch 9, wobei das Pfropfcopolymer Poly(L-lysine)-graft-poly(ethylenglykol) ist.

12. Implantat aus einem der vorangehenden Ansprüche, wobei die obere Schicht nicht kovalent an die Zwischenschicht gebunden ist.

13. Implantat aus Anspruch 1 bis 11, wobei die obere Schicht kovalent an die Zwischenschicht gebunden ist.

14. Implantat aus einem der vorangehenden Ansprüche, wobei der Linker ausgewählt ist aus den Peptiden RGD, KRSR, (KRSR)₃ und FHRIKKA, SY(SO₃)DY(SO₃), Heparin und zerstückeltem Heparin.

15. Implantat aus einem der vorangehenden Ansprüche, wobei der Linker ausgewählt ist aus der Gruppe bestehend aus Nukleinsäuren und PNS.

16. Implantat aus einem der vorangehenden Ansprüche, wobei die obere Schicht mindestens einen Träger umfasst, welcher ausgewählt ist aus der Gruppe bestehend aus Lipid-Vesikeln, Polyelektrolyt stabilisierten Vesikeln, Mikrokugeln oder Nanopartikeln.

17. Implantat aus Anspruch 16, wobei der Träger Polylactid oder Poly(lactid-co-glykolid)-Mikrokugeln umfasst.

18. Implantat aus einem der vorangehenden Ansprüche, wobei die bioaktive Substanz ausgewählt ist aus der Gruppe von systemischen Hormonen, Zytokine, Wachstumsfaktoren, Zahnschmelzmatrix-Derivaten (EMD) und entzündungshemmenden Mitteln.

19. Implantat aus Anspruch 18, wobei die bioaktive Substanz ausgewählt ist aus der Gruppe von Parathormonen, VEGF und den knochenbildungsanregenden Eiweissverbindungen.

20. Implantat aus Anspruch 19, wobei die bioaktive Substanz aus den knochenbildungsanregenden Eiweissverbindungen ausgewählt ist.

21. Implantat aus Anspruch 19, wobei die bioaktive Substanz aus den Parathormonen ausgewählt ist.

22. Implantat aus Anspruch 18, wobei die bioaktive Substanz aus den Zahnschmelzmatrix-Derivaten (EMD) ausgewählt ist.

23. Bausatz zur Herstellung des Implantats aus Anspruch 1 bis 22 umfassend (i) ein einzeln gelagertes Implantat, welches mindestens teilweise eine aufgeraute Oberfläche aufweist, (ii) zwei einzeln gelagerte Komponenten zur Bildung einer Zwischenschicht, welche verschiedene Linker ausgewählt aus der Gruppe bestehend aus Nukleinsäuren, PNS, Peptiden, Proteinen, glykosilierten Proteinen und Karbohydraten umfasst, und (iii) mindestens eine einzeln gelagerte Komponente umfassend Träger mit mindestens einer bioaktiven Substanz.

24. Bausatz aus Anspruch 21, wobei das einzeln gelagerte Implantat (i) mindestens teilweise eine hydroxylierte Oberfläche aufweist.

25. Bausatz aus Anspruch 23, wobei die Komponenten (ii) und (iii) einzeln in Behältern gelagert sind.

26. Implantat aus einem der Ansprüche 1 bis 23, wobei das Implantat ein Dentalimplantat ist.

27. Verwendung der Komponente (ii), umfassend mindestens eine einzeln gelagerte Komponente zur Bildung einer Zwischenschicht, und der Komponente (iii), umfassend mindestens eine einzeln gelagerte Komponente, welche Träger mit mindestens einer bioaktiven Substanz umfasst, um ein Implantat, welches mindestens teilweise eine aufgeraute Oberfläche aufweist, zu beschichten.

28. Implantat aus Anspruch 27, wobei das Implantat mindestens teilweise eine hydroxylierte Oberfläche aufweist.

## Revendications

1. Implant métallique comportant au moins partiellement une surface rugosifiée, laquelle est au moins partiellement recouverte par une couche intermédiaire organique ou polymère et, fixée à celle-ci, une couche supérieure avec au moins un agent bioactif et ladite couche supérieure permettant une libération contrôlée du ou des agents bioactifs, dans lequel la couche supérieure est fixée à la couche intermédiaire par un lieur sélectionné dans le groupe constitué d'acides nucléiques, d'acides nucléiques peptidiques, de peptides, de protéines, de protéines glycosylées et d'hydrates de carbone.

2. Implant selon la revendication 1, dans lequel la surface est au moins partiellement hydroxylée.

3. Implant selon l'une quelconque des revendications précédentes, l'implant métallique comprenant du titane, du zirconium, du niobium, du tantale, du hafnium ou des compositions de ceux-ci.

4. Implant selon l'une quelconque des revendications précédentes, dans lequel la surface rugueuse comprend une macro-rugosité avec une taille de cratère supérieure à 10 µm.

5. Implant selon la revendication 4, dans lequel la surface rugueuse comprend la macro-rugosité avec une taille de cratère supérieure à 20 µm.

6. Implant selon l'une quelconque des revendications précédentes, dans lequel la surface rugueuse comprend une micro-rugosité avec une taille de cratère de 4 µm ou moins.

7. Implant selon la revendication 6, dans lequel la surface rugueuse comprend la micro-rugosité avec une taille de cratère de 2 µm ou moins.

8. Implant selon l'une quelconque des revendications précédentes, l'implant étant complètement recouvert par une couche intermédiaire organique ou polymère.

9. Implant selon l'une quelconque des revendications précédentes, dans lequel la couche intermédiaire est un copolymère greffé.

10. Implant selon la revendication 6, dans lequel la couche intermédiaire est du DOPA-PEG.

11. Implant selon la revendication 9, dans lequel le copolymère greffé est le poly(L-lysine)-g-poly(éthylène glycol).

12. Implant selon l'une quelconque des revendications précédentes, dans lequel la couche supérieure est liée de façon non covalente à la couche intermédiaire.

13. Implant selon une des revendications 1 à 11, dans lequel la couche supérieure est liée de façon covalente à la couche intermédiaire.

14. Implant selon l'une quelconque des revendications précédentes, dans lequel le lieur est sélectionné parmi les peptides RGD, KRSR, (KRSR)₃ et FHRIKKA, SY(SO₃)DY(SO₃), l'héparine et l'héparine fragmentée.

15. Implant selon l'une quelconque des revendications précédentes, dans lequel le lieur est sélectionné dans le groupe constitué d'acides nucléiques et d'acides nucléiques peptidiques.

16. Implant selon l'une quelconque des revendications précédentes, dans lequel la couche supérieure comprend au moins un vecteur qui est sélectionné dans le groupe constitué de vésicules lipidiques, de vésicules stabilisées par des polyélectrolytes, de microsphères ou de nanoparticules.

17. Implant selon la revendication 16, dans lequel le vecteur comprend des microsphères de poly(acide lactique) ou de poly(lactide-co-glycolide).

18. Implant selon l'une quelconque des revendications précédentes, dans lequel l'agent bioactif est sélectionné dans le groupe constitué d'hormones systémiques, de cytokines, de facteurs de croissance, de dérivés de la matrice de l'émail (DME) et de médicaments anti-inflammatoires.

19. Implant selon la revendication 18, dans lequel l'agent bioactif est sélectionné dans le groupe constitué d'hormones parathyroïdiennes, du facteur de croissance endothélial vasculaire, et de protéines morphogénétiques osseuses.

20. Implant selon la revendication 19, dans lequel l'agent bioactif est sélectionné parmi les protéines morphogénétiques osseuses.

21. Implant selon la revendication 19, dans lequel l'agent bioactif est sélectionné parmi les hormones parathyroïdiennes.

22. Implant selon la revendication 18, dans lequel l'agent bioactif est sélectionné parmi les dérivés de la matrice de l'émail (DME).

23. Kit pour la préparation de l'implant selon une des revendications 1 à 22, comprenant (i) un implant stocké individuellement comportant au moins partiellement une surface rugosifiée, (ii) deux composants stockés individuellement pour la formation d'une couche intermédiaire comprenant différents lieurs sélectionnés dans le groupe constitué d'acides nucléiques, d'acides nucléiques peptidiques, de peptides, de protéines, de protéines glycosylées et d'hydrates de carbone, et (iii) au moins un composant stocké individuellement comprenant des vecteurs avec au moins un agent bioactif.

24. Kit selon la revendication 23, dans lequel l'implant stocké individuellement (i) comporte au moins partiellement une surface hydroxylée.

25. Kit selon la revendication 23, dans lequel les composants (ii) et (iii) sont stockés individuellement dans des contenants.

26. Implant selon l'une quelconque des revendications 1 à 23, l'implant étant un implant dentaire.

27. Utilisation du composant (ii), comprenant au moins un composant stocké individuellement pour la formation d'une couche intermédiaire, et du composant (iii), comprenant au moins un composant stocké individuellement comprenant des vecteurs avec au moins un agent bioactif, pour revêtir un implant comportant au moins partiellement une surface rugosifiée.

28. Implant selon la revendication 27, l'implant comportant au moins partiellement une surface hydroxylée.
